# EUROPEAN PATENT SPECIFICATION

(11) **EP 0 530 970 B1**
(45) Date of publication and mention of the grant of the patent: **24.01.1996**
(21) Application number: 92306912.4
(22) Date of filing: 29.07.1992
(51) Int. Cl.: A61M 25/00

(54) **Surgical introducer sheath**
Chirurgische Einführungshülse
Gaine d'introduction chirurgicale

(30) Priority: 07.08.1991 US 741689
(43) Date of publication of application: 10.03.1993
(73) Proprietor: Cook Incorporated, Bloomington Indiana 47402 (US)
(72) Inventor: Parker, Fred T., Unionville, Indiana 47468 (US)
(74) Representative: Johnston, Kenneth Graham

(56) References cited:
- EP-A- 0 144 629
- US-A- 3 879 516
- US-A- 3 956 909
- US-A- 4 044 765
- US-A- 4 516 972

## Description

This invention relates to surgical introducer sheaths.

Introducer sheaths are well-known for percutaneous vascular access such as for introducing catheters and/or medication. These sheaths are of a thin-wall construction such as of polytetrafluoroethylene or fluorinated ethylene propylene, but tend to kink. Increasing the wall thickness only minimally improves the level of kink resistance. Sheaths used in hemofiltration and dialysis may become bent or pinched off due to repeated use or patient movement. A kinked sheath cannot be straightened while in a patient, and must be removed. An alternative site if available must then be used and could cause a life threatening situation.

Such sheaths can be easily kinked whilst various catheters are introduced therethrough during emergency procedures. Small diameter introducer sheaths are also typically bent and kinked under the time constraints of an emergency situation.

Introducer sheaths are described in U.S. Patent Nos. 4,634,432; 4,657,772 and 4,705,511.

According to the present invention there is provided a surgical introducer sheath as defined in claim 1, or a method of manufacture as defined in claim 8.

The coil reinforces the wall to provide an extremely kink-resistant introducer sheath. A predetermined uniform spacing between the coils is preferable. Extremely wide spacing weakens the wall and creates a rough surface. Narrow spacing does not allow sufficient room for connecting the outer tube to the inner tube. In the preferred embodiment, the coil comprises a flat wire coil for further improving the strength of the introducer sheath.

The wall of the inner tube advantageously prevents the coil turns from extending into the inner tube passageway. As a result, the inner tube passageway has a uniform diameter for passing the largest possible diameter catheter therethrough. The protrusion of coil turns into the passageway would establish a varying diameter, which would limit the size of the catheter passable therethrough.

The heat formable polyamide material is advantageously self-levelling for providing a smooth outer surface which also reduces the formation of blood clots thereon.

The inner diameter of the passageway about the distal ends of the inner and outer tubes is uniform to again minimize the formation of blood clots on the inner surface of the inner tube.

### Brief description of the drawings

FIG.1 depicts an embodiment introducer sheath;
FIG.2 is a partially sectioned view of FIG.1;
FIGs.3 and 4 are views illustrating manufacture of the sheath.

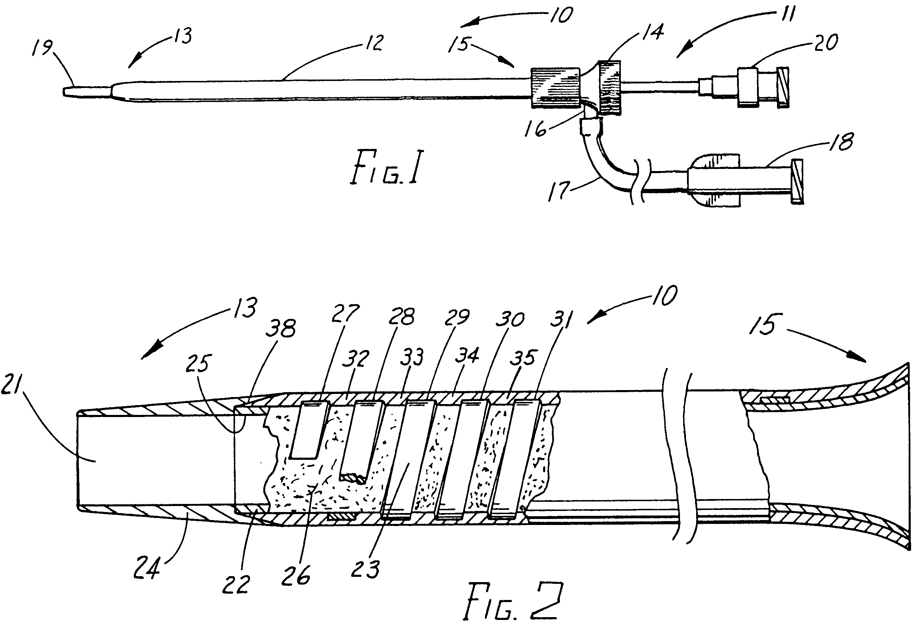

FIG.1 depicts an illustrative flexible, kink-resistant, introducer sheath 10 with a tapered dilator 11 extending longitudinally through the passageway of the sheath. As shown, the introducer sheath includes an outer tube 12 with a tapered distal end 13 and connector valve 14 attached about proximal end 15 of the sheath. Valve 14 includes a silicone disk (not shown) for preventing the backflow of fluids. The disk includes a slit for the insertion of dilator 11. By way of example, the dilator 11 has a 6.0 French 0.2cms (0.079") outside diameter. Connector 14 also includes side arm 16 to which polyvinyl tube 17 and male Luer lock connector 18 are connected for introducing and aspirating fluids therethrough. Dilator 11 includes tapered distal end 19 for accessing and dilating a vascular access site over a well-known and commercially available wire guide. The guide is inserted in the vessel with an introducer needle using, for example, the well-known percutaneous vascular access Seldinger technique. A well-known male Luer lock connector hub 20 is attached at the proximal end of the dilator for connection to syringes and other medical apparatus.

Depicted in FIG.2 is a partially sectioned view of introducer sheath 10 with dilator 11 removed from longitudinal passageway 21. The sheath comprises inner tube 22, flat wire reinforcing coil 23 compression fitted therearound, and outer tube 12 mechanically connected to roughened outer surface 26 of the inner tube through the spacings of the coil. Inner tube 22 is a 7.4cms length of a lubricous material tube such as polytetrafluoroethylene having a uniform inside diameter in the range of 0.2cms (0.0825") to 0.213cms (0.084") with a wall thickness of 0.0038cms (0.0015") plus or minus 0.0013 cms (0.0005") before heating. The inner tube has a minimum inside dimension of 0.206 cms (0.081") after heating. The lubricous polytetrafluoroethylene material presents a slippery inner surface 25 for the easy insertion and withdrawal of the dilator as well as other catheters and medical apparatus. Inner surface 25 is also smooth and nonporous for minimizing the formation of blood clots and other thrombi thereon. Outer surface 26 of the inner tube is chemically etched to form a rough outer surface to which outer tube 12 is mechanically connected using a shrinking and formation process. The uniform inner diameter of inner tube 22 extends the entire length of passageway 21 for passing the largest possible diameter catheter therethrough. The wall of the inner tube prevents the turns of compression-fitted coil 23 from protruding into inner tube passageway 21.

Coil 23 comprises a plurality of spaced flat wire turns such as 27-31, which preferably have equal width spaces 32-35 therebetween. Coil 23 is 6.5cms in length with an outside diameter of 0.239cms (0.0942") plus or minus 0.005cms (0.002") formed from 0.0076cms (0.003") thick by 0.03cms (0.012") wide flat rectangular stainless steel wire wound preferably with a uniform space in the range of 0.013cms (0.005") to 0.038cms (0.015") between the turns of the coil. Wire coil 23 is compression fitted around the outer surface of inner tube 22 approximately 4mms from the distal end thereof and approximately 5mms from the proximal end thereof to maintain the spacing between the turns of the coil. The coil is compression fitted by collapsing inner tube 22 and inserting the wire coil thereover. Inner tube 22 is then compressed-air expanded to engage and compression fit the inner surface of the flat wire coil. A mandril inserted through the passageway of the inner tube further compresses the inner tube against the coil turns during the manufacture of the sheath as hereinafter described. The coil is preferably positioned away from the distal and proximal ends of the inner tube to permit tapering and flaring of the sheath without extending the coil turns through the polyamide material of the outer tube.

Outer tube 12 is 7.4cms in length with an inside diameter of 0.26cms (0.103") plus or minus 0.005cms (0.002") of a heat formable polyamide material such as nylon that is heat shrunk over coil 23, which in turn is compression fitted over inner tube 22. The wall thickness of the nylon tube is approximately 0.0165cms (0.0065") plus or minus 0.00254cms (0.001"). The outer tube is heated and compressed through the spaces between the coil turns with a heat shrink tube for mechanically connecting to rough outer surface 26 of the inner tube. As a result, the outside diameter of the outer tube is approximately 0.0559cms (0.022") greater than that of the inner tube. After the outer tube is heat shrunk on to the roughened surface of the inner tube, the shrink tube is removed therefrom, and a taper formed at the distal end of the sheath. As a result, the thickness of the sheath including the inner tube, coil and outer tube is approximately 0.0279cms (0.011"). The 4mms length about the distal end of the inner and outer tubes are cut to within a range of 0.0254cms (0.01") to 0.229cms (0.09") from the end of coil 23 depending on the inside diameter of the sheath. For a 6.0 French introducer sheath, approximately 0.0508cms (0.02") of outer tube 12 is externally tapered about the distal end to form contact surface area 38. Tapered distal end 13 is formed by cutting and slitting a 3mms length of nylon tubing having a 0.254cms (0.1") inside diameter and inserting it into a taper mould. The short length of tubing is heated, and the distal end of the sheath with a mandril inserted therethrough is inserted into the taper mould to thermally bond nylon tip material 24 to the outer tube and to form tapered distal end 13, as shown. As a result, the inside diameter of outer tube 12 and inner tube 22 about the distal end thereof assumes the uniform inner diameter of the inner tube. After the distal end is tapered, the outer tube extends approximately 0.3cms (0.12") beyond the distal end of the inner tube and 0.356cms (0.14") beyond the distal end of the flat wire coil. The distal end of inner tube 22 may vary along the length of the tapered distal end of the outer tube, but should not extend all the way to the distal end of the outer tube so as not to break the tapered surface of the outer tube. In this particular embodiment, nylon tip material 24 is of the same durometer as that of outer tube 12. However, it is contemplated that the tip material may have a durometer other than that of the outer tube material. It is further contemplated that the tip material preferably has a harder durometer so as to further facilitate entry into the access site. It can, however, be softer if required. Proximal end 15 of the sheath is formed into a flared configuration such as inserting over a heated, tapered tip end and then cooled.

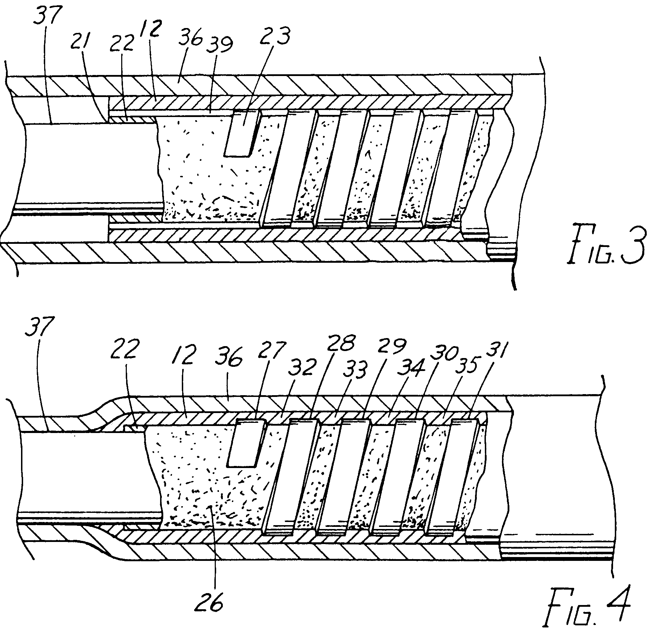

FIG.3 depicts a partially sectioned view of introducer sheath 10 with heat shrink tube 36 positioned over outer tube 12 and flat wire coil 23 with longitudinal space 39 therebetween. As previously described, flat wire coil 23 is compression fitted around inner tube 22. Prior to heating shrink tube 36 and forming outer tube 12, mandril 37 is inserted through passageway 21 to preferably maintain uniform spacing between the coil turns. As shown, heat shrink tube 36 is somewhat longer than nylon outer tube 12 and has an inside diameter in the range of 0.21cms (0.0825") to 0.213cms (0.084") with a wall thickness of approximately 0.0038cms (0.0015") plus or minus 0.0013cms (0.0005"). The heat shrink tube is preferably of a fluorinated ethylene propylene heat formable material . The nylon outer tube has a processing temperature range for the heat formation thereof in the range of 198°C to 278°C (356 to 500 degrees Fahrenheit).

FIG.4 depicts heat shrink tube 36 being oven heated to a temperature of 203°C (365 degrees Fahrenheit), which is in the processing temperature range of the nylon outer tube material. As the heat shrink tube shrinks, the heated nylon outer tube material 12 is compressed between coil turns 27-31 in spaces 32-35 to mechanically connect with roughened surface 26 of inner tube 22. The heat formable nylon material tube is also self-levelling, which provides a uniform outer diameter surface for the sheath. Heat shrink tube 36 is then split from the sheath. As previously described, distal end 13 is tapered, and proximal end 15 is flared.

It is contemplated that various other materials may be utilized for the inner, outer and heat shrink tubes. It is also contemplated that introducer sheaths with an inside diameter ranging in size from 5.5 to 14.0 French are readily producible. In summary, the flexible, kink-resistant introducer sheath provides a thin-wall sheath that is extremely kink-resistant for long-term use applications. The flat wire coil construction of this introducer sheath is also extremely kink-resistant with small outside diameter dilators during introduction through an access site.

## Claims

1. A flexible, kink-resistant, surgical introducer sheath comprising tubular material (12,22) with a reinforcing coil (23) therein, said tubular material having a passageway (21) extending longitudinally therethrough, characterised in that the tubular material comprises an inner tube (22) and an outer tube (12), in that the coil has spaced turns (27 to 31 etc.) formed about the inner tube, and in that the inner surface of the outer tube is formed about and in contact with the coil and also in contact with the inner tube between the spaces of the said turns.

2. The sheath of claim 1, characterised in that the coil has equally spaced turns.

3. The sheath of claim 1 or 2, characterised in that the turns are of flat wire.

4. The sheath of claim 1, 2 or 3, characterised in that the ends of the inner and outer tubes extend beyond the ends of the coil.

5. The sheath of claim 4, characterised in that the distal end of the outer tube extends beyond the distal end of the inner tube, and is tapered, and/or tip material (24) is bonded to the distal end of the outer tube, said tip material being designed to facilitate sheath insertion.

6. The sheath of any one preceding claim, characterised in that the sheath has a uniform internal diameter; and/or the proximal end of the tube is flared.

7. The sheath of any one preceding claim, characterised in that the inner tube has a smooth inner surface, and/or the inner tube comprises a lubricous material such as polytetrafluoroethylene, and/or the outer tube comprises polyamide which may comprise nylon.

8. A method of manufacturing a percutaneous introducer sheath, said method comprising the step of securing a reinforcing coil (23) within tubular material(s) (12,22), said tubular material(s) defining a passageway (21) extending longitudinally therethrough; characterised in that the said sheath comprises an inner tube (22) and an outer tube (12); in that the coil is compression fitted around the inner tube; and in that the outer tube is heat treated to extend between the spaces of the coil and bond to the inner tube.

9. The method of claim 8, characterised in that the inner tube has a roughened outer surface, and/or a smooth inner surface, and/or the turns of the coil are of predetermined spacing.

10. The method of claim 8 or 9, characterised in that a shrinkable tube (36) is positioned around the outer tube in order to compress the outer tube between the spaces of the said turns, and/or a mandril (37) is positioned inside the inner tube to maintain the shape of the sheath during manufacture.

## Patentansprüche

1. Flexibler, knickfester, chirurgischer Einführschaft, der rohrförmiges Material (12, 22) mit einer darin enthaltenen Verstärkungsspule (23) umfaßt, wobei das rohrförmige Material einen sich längs durch es hindurch erstreckenden Durchgang (21) aufweist, dadurch gekennzeichnet, daß das rohrförmige Material ein Innenrohr (22) und ein Außenrohr (12) umfaßt, daß die Spule um das Innenrohr herum gebildete, voneinander beabstandete Windungen (27 bis 31 usw.) aufweist und daß die Innenfläche des Außenrohrs um die Spule herum und diese berührend sowie das Innenrohr in den Zwischenräumen zwischen den Windungen berührend gebildet ist.

2. Schaft nach Anspruch 1, dadurch gekennzeichnet, daß die Spule gleichmäßig beabstandete Windungen aufweist.

3. Schaft nach Anspruch 1 oder 2, dadurch gekennzeichnet, daß die Windungen aus flachem Draht bestehen.

4. Schaft nach Anspruch 1, 2 oder 3, dadurch gekennzeichnet, daß die Enden der Innen- und Außenrohre sich über die Enden der Spule hinaus erstrecken.

5. Schaft nach Anspruch 4, dadurch gekennzeichnet, daß das distale Ende des Außenrohrs sich über das distale Ende des Innenrohrs hinaus erstreckt und sich verjüngt, und/oder daß Spitzenmaterial (24) mit dem distalen Ende des Außenrohrs verbunden ist, wobei das Spitzenmaterial so ausgebildet ist, daß es die Einführung des Schaftes erleichtert.

6. Schaft nach einem der vorhergehenden Ansprüche, dadurch gekennzeichnet, daß der Schaft einen gleichmäßigen Innendurchmesser aufweist, und/oder daß das proximale Ende des Rohrs aufgeweitet ist.

7. Schaft nach einem der vorhergehenden Ansprüche, dadurch gekennzeichnet, daß das Innenrohr eine glatte Innenfläche aufweist, und/oder daß das Innenrohr ein Gleitmaterial wie Polytetrafluorethylen aufweist, und/oder daß das Außenrohr aus Polyamid ist, wobei es sich auch um ein Nylon handeln kann.

8. Verfahren zur Herstellung eines perkutanen Einführschaftes, bei dem man eine Verstärkungsspule (23) in dem (den) rohrförmigen Material(ien) (12, 22) befestigt, wobei jene(s) rohrförmige(n) Material(ien) einen sich längs durch es (sie) hindurch erstreckenden Durchgang (21) definiert(en), dadurch gekennzeichnet, daß der Schaft ein Innenrohr (22) und ein Außenrohr (12) umfaßt und daß man die Spule stauchend über das Innenrohr steckt und das Außenrohr wärmebehandelt, damit es sich durch die Zwischenräume der Spule erstreckt und sich mit dem Innenrohr verbindet.

9. Verfahren nach Anspruch 8, dadurch gekennzeichnet, daß das Innenrohr eine aufgerauhte Außenfläche und/oder eine glatte Innenfläche aufweist, und/oder daß die Windungen der Spule einen vorherbestimmten Abstand aufweisen.

10. Verfahren nach Anspruch 8 oder 9, dadurch gekennzeichnet, daß ein schrumpffähiges Rohr (36) um das Außenrohr herum positioniert wird, um das Außenrohr in die Zwischenräume der Windungen zusammenzudrücken, und/oder daß ein Dorn (37) im Innenrohr positioniert wird, um die Form des Schaftes während der Herstellung aufrechtzuerhalten.

## Revendications

1. Gaine d'introduction chirurgicale, flexible, résistant au tortillement comprenant un matériau tubulaire (12, 22) avec un boudin de renforcement (23) à l'intérieur, ledit matériau tubulaire présentant un passage (21) gui s'étend longitudinalement à travers, caractérisée en ce que le matériau tubulaire comprend un tube interne (22) et un tube externe (12), en ce que le boudin comprend des spires espacées (27 à 31, etc.) formées autour du tube interne et en ce que la surface interne du tube externe est formée autour du boudin et est en contact avec le boudin et avec le tube interne entre les espaces desdites spires.

2. Gaine suivant la revendication 1, caractérisée en ce que le boudin comprend des spires espacées de manière égale.

3. Gaine suivant la revendication 1 ou 2, caractérisée en ce que les spires sont faites de fil métallique plat.

4. Gaine suivant la revendication 1, 2, ou 3, caractérisée en ce que les extrémités des tubes interne et externe s'étendent au-delà des extrémités du boudin.

5. Gaine suivant la revendication 4, caractérisée en ce que l'extrémité distale du tube externe s'étend au-delà de l'extrémité distale du tube interne, et est conique, et/ou un matériau d'embout (24) est relié à l'extrémité distale du tube externe, ledit matériau d'embout étant destiné à faciliter l'introduction de la gaine.

6. Gaine suivant l'une quelconque des revendications précédentes, caractérisée en ce que la gaine a un diamètre interne uniforme; et/ou l'extrémité proximale du tube est évasée.

7. Gaine suivant l'une quelconque des revendications précédentes, caractérisée en ce que le tube interne présente une surface interne lisse, et/ou le tube interne comprend un matériau lubrifiant tel que le polytétrafluoroéthylène, et/ou le tube externe comprend un polyamide qui peut comprendre du Nylon.

8. Procédé de fabrication d'une gaine d'introduction percutanée, ledit procédé comprenant l'étape consistant à fixer un boudin de renforcement (23) dans le(s) matériau(x) tubulaire(s) (12, 22), le(s)dit(s) matériau(x) tubulaire(s) définissant un passage (21) s'étendant longitudinalement à travers; caractérisé en ce que ladite gaine comprend un tube interne (22) et un tube externe (12); en ce que le boudin est monté à compression autour du tube interne; et en ce que le tube externe est traité thermiquement afin de s'étendre entre les espaces du boudin et d'être en contact avec le tube interne.

9. Procédé suivant la revendication 8, caractérisé en ce que le tube interne possède une surface externe rugueuse, et/ou une surface interne lisse, et/ou les spires du boudin sont espacées de manière prédéterminée.

10. Procédé suivant la revendication 8 ou 9, caractérisé en ce qu'un tube rétractable (36) est positionné autour du tube externe de manière à comprimer le tube externe entre les espaces desdites spires, et/ou un mandrin (37) est placé à l'intérieur du tube interne afin de maintenir la configuration de la gaine pendant la fabrication.
